# FASCICULE DE BREVET EUROPEEN

(11) **EP 1 765 154 B1**
(45) Date de publication et mention de la délivrance du brevet: **25.02.2009**
(21) Numéro de dépôt: 05772963.4
(22) Date de dépôt: 25.05.2005
(51) Int. Cl.: A61B 5/01, G01J 5/00

(54) **DISPOSITIF POUR LA MESURE A DISTANCE DE LA TEMPERATURE D'UN OBJET OU D'UN CORPS VIVANT**
VORRICHTUNG ZUR TEMPERATURFERNMESSUNG EINES OBJEKTS ODER LEBENDEN KÖRPERS
DEVICE FOR REMOTE MEASUREMENT OF THE TEMPERATURE OF AN OBJECT OR LIVING BODY

(30) Priorité: 25.05.2004 FR 0405639
(43) Date de publication de la demande: 28.03.2007
(73) Titulaire: Realtrace, 91140 Villebon sur Yvette (FR)
(72) Inventeur: BIMBARD, Franck, F-94800 Villejuif (FR); FINET, Paul, F-91140 Villebon sur Yvette (FR); MICHOT, Gérard, F-78720 Dampierre (FR)
(74) Mandataire: de Saint-Palais, Arnaud Marie
(86) Numéro de dépôt international: PCT/FR2005/001290
(87) Numéro de publication internationale: WO 2005/117689

(56) Documents cités:
- EP-A- 0 270 299
- EP-A- 0 594 102
- DE-C1- 3 710 486
- FR-A- 2 772 918
- FR-A- 2 801 491
- US-A- 4 379 461
- US-A- 4 494 881
- US-A- 4 634 294
- US-A- 4 722 612
- US-A1- 2002 010 390
- US-A1- 2003 099 277
- US-A1- 2003 128 735
- US-B1- 6 346 885
- HARIGOVINDAN S ET AL: "REMOTE-SENSING INFRARED THERMOMETER WITH RADIATION BALANCING" APPLIED OPTICS, OPTICAL SOCIETY OF AMERICA,WASHINGTON, US, vol. 39, no. 15, 20 mai 2000 (2000-05-20), pages 2461-2466, XP000950196 ISSN: 0003-6935

## Description

La présente invention concerne un dispositif pour la mesure à distance de la température d'un objet ou d'un corps vivant.

Elle s'applique notamment, mais non exclusivement, à la mesure de la température d'un animal avec identification à distance de cet animal.

D'une façon générale, on sait que la prise de la température de bon nombre d'animaux, selon la méthode traditionnelle, pose de réels problèmes que connaissent bien les vétérinaires :
- soit en raison des risques de blessures (morsures, griffures) qu'encourt l'opérateur,
- soit en raison de la réaction de l'animal au stress que lui procure cette opération, réaction qui se traduit par une élévation de la température, de sorte que la température mesurée n'est plus significative.

Pour tenter de surmonter ces problèmes, on a déjà proposé plusieurs solutions.

L'une de ces solutions consiste à incorporer à un transpondeur inséré sous la peau, un capteur de température de manière à pouvoir faire une lecture à distance de cette température au moyen d'un dispositif de lecture approprié.

Toutefois, cette solution pose de nombreux problèmes techniques qui, à ce jour, sont mal résolus. En outre, cette solution conduit à l'acquisition de la température en un point indéterminé de l'animal. Or, la température à la superficie du corps d'un animal n'est pas constante de sorte que le résultat de la mesure n'est pas forcément représentatif de la température moyenne de ce corps.

Une autre solution consiste à mesurer le rayonnement infrarouge émis par une zone du corps de l'animal de la même façon que celle qui est actuellement utilisée pour des êtres humains (mesure des radiations IR émises dans le conduit externe de l'oreille). Il s'avère que pour les humains, cette méthode est contestée et que le résultat obtenu nécessite d'être corrigé pour obtenir la température moyenne du corps. Ceci est dû notamment aux différences morphologiques des oreilles et des variations de surface des zones émissives de rayons infrarouges. En outre, cette solution convient mal pour les animaux présentant une oreille interne très profonde et dont les conduits auditifs externes sont souvent protégés par des oreilles qu'il faut soulever et par une pilosité qui perturbe la propagation des ondes infrarouges.

Le brevet US 4 494 881 décrit un dispositif pour la mesure à distance de la température correspondant au préambule de la revendication 1.

L'invention a donc plus particulièrement pour but la réalisation d'un dispositif de mesure à distance de température qui ne présente pas les inconvénients précités, ce dispositif convenant pour la prise de température d'une grande variété d'animaux et permettant de fournir une valeur de température correspondant à la température moyenne du corps de l'individu ou de l'animal.

Elle propose de parvenir à ce résultat en mesurant le rayonnement émis par une zone du corps de dimension prédéterminée ou mesurée au moment de la mesure de température et en prenant en compte les éléments susceptibles de modifier le résultat de la mesure tel que l'angle d'incidence, la température ambiante et le milieu dans lequel se propage le rayonnement infrarouge, notamment le taux d'atténuation du milieu dans lequel se propage ce rayonnement.

A cet effet, ce dispositif fait intervenir un capteur infrarouge directionnel présentant un volume de détection cylindrique ou légèrement conique de manière à détecter le rayonnement infrarouge émis exclusivement par une première zone de la surface émissive de l'objet ou du corps dont on veut mesurer la température, qui se trouve incluse dans ledit volume de détection ainsi que des moyens de traitement des informations délivrées par le capteur qui coopèrent avec des moyens de calcul pour déterminer la température dudit objet ou dudit corps à partir desdites informations.

Selon l'invention, ce dispositif comprend en outre une source de rayonnement lumineux émettant dans le spectre visible, cette source étant associée à des moyens de projection engendrant un faisceau lumineux apte à éclairer une deuxième zone de la surface émissive incluant la première zone.

Le dispositif comprend également un transmetteur directionnel qui rayonne dans un domaine précis de longueur d'onde au voisinage de l'axe du capteur et des moyens permettant de déterminer un paramètre relatif à l'atténuation que subit ce rayonnement au cours de son trajet vers la zone émissive, de sa réflexion sur ladite zone et de son retour à la partie réception du transmetteur.

Le paramètre ainsi déterminé est alors utilisé pour calculer la correction à apporter à la valeur de température mesurée par le capteur.

Pour éviter que cet éclairement ne produise une élévation notoire de la température de la zone émissive, le susdit éclairement pourra être engendré par une source lumineuse impulsionnaire permettant d'obtenir une lumière « froide » et de longueur d'onde éloignée de celle du rayonnement IR de la zone émissive.

Dans le cas où le volume de détection est conique et où les dimensions de la première zone varient de façon importante en fonction de la distance entre le capteur et la surface émissive, le calcul de la température devra prendre en compte cette distance. Dans ce cas, il conviendra de prévoir des moyens permettant de mesurer la distance entre le capteur et la zone émissive. Avantageusement ces moyens pourront consister en une toise ou en des moyens de télémétrie pouvant faire intervenir un émetteur/récepteur et une électronique de comptage du temps que met une impulsion émise par l'émetteur pour parvenir au récepteur après réflexion sur la surface émissive. Eventuellement, l'électronique de comptage pourra être couplée à des moyens de signalisation émettant un signal d'avertissement lorsque la distance souhaitée est atteinte.

Par ailleurs, un capteur de température intégré ou non au dispositif pourra être prévu pour mesurer la température ambiante.

La valeur de la température ambiante ainsi mesurée, pourra être utilisée dans le calcul de la susdite correction.

Des modes d'exécution de l'invention seront décrits ci-après, à titre d'exemples non limitatifs, avec référence aux dessins annexés dans lesquels :
La figure 1 est une représentation schématique d'un dispositif selon l'invention utilisant un faisceau de localisation coaxial au capteur de rayonnement IR ;
La figure 2 est un schéma synoptique du circuit d'asservissement en température du capteur de rayonnement IR ;
La figure 3 est une vue agrandie du dispositif de la figure 1 montrant schématiquement les moyens électroniques utilisés ;
La figure 4 est une représentation similaire à celle de la figure 2, dans laquelle le faisceau de localisation est décalé par rapport à l'axe du capteur de rayonnement IR.

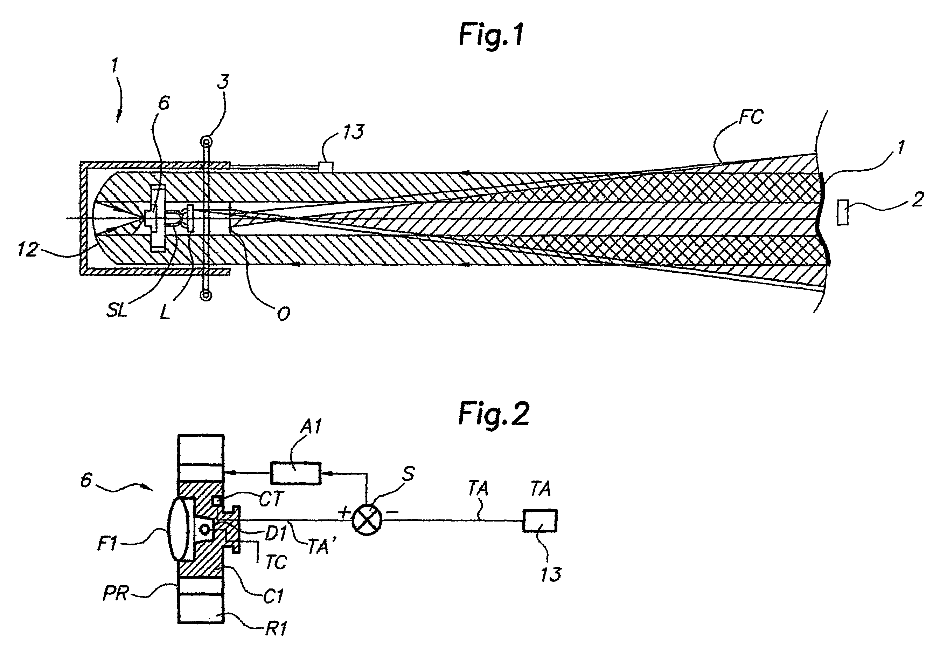

Le dispositif illustré sur la figure 1 est destiné à l'identification et à la mesure à distance de la température d'un animal dans une zone 1, schématiquement représentée sur la figure 1. Dans cet exemple, une puce transpondeur 2 a été implantée dans cette zone, sous l'épiderme de l'animal.

Cette puce transpondeur 2, de type inductif RFID, comprend un circuit électronique comportant une mémoire permettant de mémoriser des données d'identification de l'animal. Ce circuit électronique comprend en outre un bobinage destiné à la fois à assurer la transmission à distance des informations contenues dans la mémoire ainsi que la réception de l'énergie électrique nécessaire au fonctionnement du circuit électronique.

Bien entendu, la présence de cette puce transpondeur 2 dans ou au voisinage de cette zone n'est pas indispensable : l'identification de l'animal pourrait être réalisée autrement (saisie informatique, lecture d'un badge etc....).

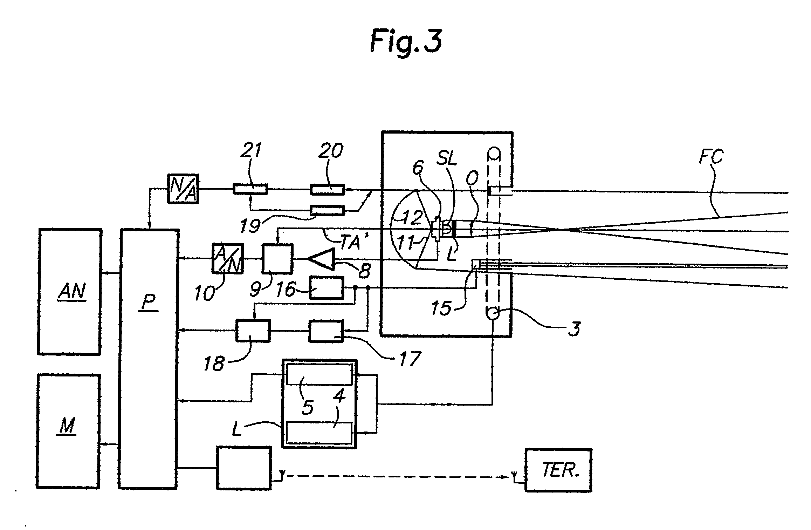

Pour effectuer la lecture de cette puce transpondeur 2, le dispositif D est équipé d'un circuit de lecture RFID L faisant intervenir une bobine 3 émettrice/réceptrice reliée à un circuit d'alimentation 4 débitant un courant alternatif dont la fréquence correspond à la fréquence de résonance du circuit de la puce 2 et un circuit de réception 5 qui assure une démodulation du signal retransmis par le bobinage de la puce 2 de manière à en extraire les données d'identification (fig. 3).

Ces données d'identification sont ensuite transmises à un processeur P et stockés en mémoire (mémoire M).

La détection de température par ce dispositif s'effectue au moyen d'un capteur de rayonnement infrarouge 6 comprenant, comme illustré figures 1 et 2, un corps C1, une optique focalisatrice F₁ et une cellule optoélectronique D1 placée au foyer de l'optique F1.

Il comprend en outre un capteur additionnel de température CT du corps C1. Dans les montages usuels, la température du corps C1 est censée représenter la température ambiante TA' et le circuit électronique associé au capteur 6 est conçu de manière à effectuer une correction de la température TC mesurée par la cellule D1 en fonction de cette température ambiante TA'.

Or il s'avère que dans un appareil portatif la température TA' du corps C1 du capteur 6 est souvent influencée par des éléments extérieurs et notamment par la température de la main de l'opérateur qui tient l'appareil. De ce fait, la température TA' détectée par le capteur CT n'est pas toujours représentative de la température ambiante.

C'est la raison pour laquelle dans cet exemple, il est prévu un système de maintien du corps C1 à la température ambiante, ce système faisant intervenir, comme illustré figure 2, une cellule à effet Peltier PR monté sur le corps C1, de manière à pouvoir abaisser la température de ce dernier. La commande de cette cellule PR est assurée par un asservissement mesurant grâce à un soustracteur S l'écart de température entre la température détectée TA' par le capteur CT et la température TA détectée par un capteur additionnel 13 placé en un endroit convenable pour mesurer la température ambiante. Le résultat de cette soustraction est alors appliqué à un amplificateur A1 qui assure la commande de la cellule à effet Peltier PR. (Ce système part du principe que les perturbations extérieures engendrant des variations de température du corps C1 provoquent un échauffement de celui-ci par rapport à la température ambiante. Les calories extraites du corps C1 par la cellule PR sont dissipées par un radiateur R1 par exemple en aluminium.

En alternative avec la solution illustrée sur les figures 1 et 2, il est possible de prévoir une microturbine engendrant sur le corps un flux d'air à la température ambiante : Cette solution permet d'éviter éventuellement l'usage d'un asservissement en température.

Comme illustré figure 3, le signal TC délivré par la cellule D1 du capteur de température 6 est amplifié dans un amplificateur 8 et passe par un circuit correcteur 9 avant d'être transmis à un convertisseur analogique/numérique 10. Le signal numérique délivré par ce convertisseur 10 est ensuite transmis à une entrée du processeur P où il est traité en vue de la détermination de la température de l'animal avant d'être stocké en mémoire M avec les données d'identification fournies par le circuit de lecture L (fig.3).

Dans cet exemple, le capteur 6 est placé sensiblement au foyer d'un réflecteur parabolique 12 disposé de manière à obtenir un volume de détection faiblement conique (dont la directrice est faiblement inclinée par rapport à l'axe du réflecteur 12).

Pour effectuer une mesure correcte de la température de l'animal, il est tout d'abord nécessaire d'amener la région émissive qui est significative de la température moyenne de l'animal, dans le champ de détection du capteur.

A cet effet, le dispositif selon l'invention comprend des moyens d'émission d'un rayonnement lumineux visible, par exemple coloré, permettant de visualiser une zone du corps de l'animal par laquelle passe le champ de détection du capteur.

Selon ce mode d'exécution, ces moyens d'émission font intervenir, disposés coaxialement au réflecteur parabolique 12 entre le capteur directionnel 6 et la surface émissive :
- une source lumineuse SL éventuellement dotée d'une lentille convergente,
- un objectif O pouvant comprendre une ou plusieurs lentilles,
- des moyens de formation d'un objet lumineux L disposés entre la source SL et l'objectif O.

Ces moyens d'émission engendrent un faisceau lumineux sensiblement conique FC destiné à produire sur la surface émissive de rayonnement IR une zone lumineuse dans laquelle se distingue l'image nette de l'objet lumineux L lorsque que la distance entre l'objectif et cette surface émissive correspond à une distance de mise au point prédéterminée. A cette distance, la surface de détection du capteur sur la zone émissive 1 est totalement incluse dans la zone lumineuse engendrée par les moyens d'émission.

La détermination de la température de l'animal à partir des informations fournies par le capteur 6 exige la connaissance de paramètres tels que :
- la température ambiante,
- la distance entre le capteur 6 et la zone émissive 1 de l'animal se trouvant dans le volume de détection V,
- la surface de cette zone émissive 1,
- l'angle d'incidence (si cet angle d'incidence est différent de l'incidence normale),
- le coefficient d'atténuation de l'onde IR dans son parcours entre la zone émissive 1 et le capteur 6.

La température ambiante est de nature à affecter la mesure réalisée par le capteur 6. Pour s'affranchir de ce problème, on utilise le signal TA' délivré par le capteur CT pour commander le circuit de correction 9. Eventuellement, ce résultat pourra être transmis au processeur P après conversion anologique / numérique. Dans ce cas, une table de correction préalablement mémorisée pourra être utilisée.

La distance D entre le capteur 6 et la zone émissive 1 peut être déterminée grâce à la mise au point précédemment décrite. Néanmoins elle pourrait être obtenue au moyen d'une toise T parallèle à l'axe du capteur 6 et présentant une longueur prédéterminée. Cette toise T peut être conçue de manière à être amovible, dépliable voire même télescopique.

La correction effectuée par le processeur P peut alors tenir compte de cette longueur.

Dans le cas où l'on souhaite pouvoir faire varier cette longueur, il est possible d'utiliser un télémètre, par exemple du type illustré sur la figure 3 qui comprend un transmetteur 15 raccordé à un circuit émetteur 16 qui émet des impulsions (radioélectriques ou optiques), à un circuit récepteur 17 qui reçoit les échos réfléchis par la zone émettrice ZE. Une électronique de comptage 18 permet de mesurer la période de temps entre l'émission de chaque impulsion et la réception de l'écho correspondant à cette impulsion. Cette période de temps est utilisée par le processeur P pour calculer la distance D. Des moyens pourront être utilisés pour informer l'utilisateur quand une distance préconisée est atteinte.

La surface de détection du dispositif sur la région émissive 1 de l'animal est déterminée en fonction de la distance D précédemment calculée, de l'angle du cône de détection et de l'angle d'incidence. Dans le cas où l'incidence n'est pas normale, des moyens de détection de cet angle d'incidence peuvent être prévus.

Le paramètre relatif à l'atténuation de l'onde IR dans son parcours entre le capteur 6 et la zone émissive ZE est obtenu grâce à un circuit transmetteur comportant une partie émettrice 19 qui émet des trains d'ondes calibrés dans un domaine précis de longueur d'onde, et une partie réceptrice 20 destinée à recevoir les ondes d'échos réfléchies par la zone émissive ZE. Le signal reçu par la partie réceptrice 20 est comparé à celui émis par la partie émettrice au niveau d'un comparateur 21. Le résultat de cette comparaison est transmis au processeur P par l'intermédiaire d'un convertisseur analogique/numérique 22.

Le processeur est programmé de manière à corriger le signal en fonction des informations émanant du transmetteur 19, 20 et du télémètre TM et éventuellement du détecteur de température ambiante 13. A cet effet, ce processeur P peut utiliser une table de calibration préalablement mémorisée.

Par ailleurs, le processeur P est conçu de manière à exécuter diverses opérations et algorithmes mathématiques, afin de présenter un résultat numérique fiable facilement interprétable sur un afficheur numérique AN.

Une temporisation des divers éléments capteur, transmetteur et éventuellement transpondeur, est orchestrée par le processeur de manière à provoquer un affichage de la température pendant une durée paramétrable après détermination.

Ces données associées (identifiant/température) pourront être transmises simultanément avec des données d'horodatage grâce à un émetteur radio E (par exemple 433 Mhz, bluetooth, etc....) à un terminal de communication 23, par exemple du type PC, téléphone, PDA, etc.

Bien entendu, l'invention ne se limite pas au mode d'exécution précédemment décrit.

Ainsi, par exemple, le volume de détection du capteur infrarouge n'est pas forcément coaxial au faisceau lumineux servant à la localisation de la zone émissive ZE se trouvant dans le champ du capteur 6. En effet, elle peut être située à côté du capteur.

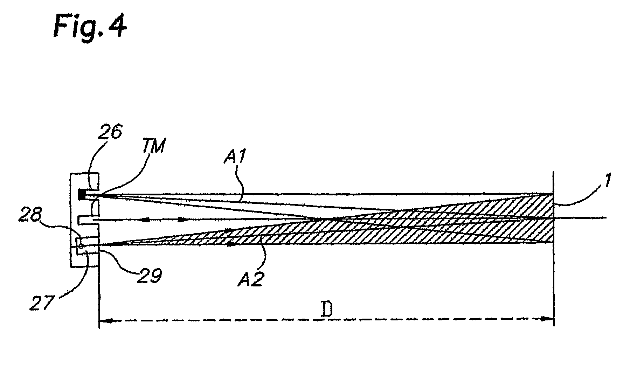

Comme illustré sur la figure 4, le capteur infrarouge 26 et les moyens d'émission 27 utilisés pour visualiser la zone émissive ZE pourront être décalés avec des axes A1, A2 sécants à une distance D appropriée à la mesure. Dans ce cas, le télémètre TM pourra être axé selon la médiatrice du triangle délimitée par les deux axes sécants A1, A2 et le capteur 26 et les moyens d'émission 27.

Dans cet exemple, les moyens d'émission sont constitués par une source lumineuse 28 focalisée par une lentille convexe 29 de manière à obtenir un faisceau lumineux présentant sensiblement la même conicité que le volume de détection du capteur. La surface éclairée représente ici la zone émissive de mesure.

## Revendications

1. Dispositif pour la mesure à distance de la température d'un objet ou d'un corps vivant, ce dispositif comprenant un capteur infrarouge directionnel (6) présentant un volume de détection cylindrique ou légèrement conique de manière à détecter le rayonnement infrarouge émis exclusivement par une première zone (1) de la surface émissive de l'objet ou du corps dont on veut mesurer la température, qui se trouve incluse dans ledit volume de détection, des moyens de traitement des informations délivrées par le capteur (6), des moyens de calcul aptes à déterminer la température dudit objet ou dudit corps à partir desdites informations, ainsi qu'une source de rayonnement lumineux (SL) apte à emettre dans le spectre visible; cette source (SL) étant associée à des moyens de projection engendrant un faisceau lumineux apte à éclairer une deuxième zone de la surface émissive incluant la première zone (1),
**caractérisé en ce qu'**il comprend un transmetteur directionnel apte à rayonner dans un domaine précis de longueur d'onde au voisinage de l'axe du capteur (6) et des moyens permettant de déterminer un paramètre relatif à l'atténuation que subit ce rayonnement au cours de son trajet vers la zone émissive (1), de sa réflexion sur ladite zone (1) et de son retour à la partie réception du transmetteur, les moyens de calcul étant conçus de manière à calculer la correction à apporter à la valeur de température mesurée par le capteur en fonction du paramètre ainsi déterminé.

2. Dispositif selon la revendication 1,
**caractérisé en ce que** la susdite source lumineuse (SL) est impulsionnaire.

3. Dispositif selon l'une des revendications 1 et 2,
**caractérisé en ce qu'**il comprend des moyens permettant de mesurer la distance (D) entre le capteur (6) et la zone émissive (ZE) ainsi que des moyens de calcul de température prenant en compte cette distance (D).

4. Dispositif selon la revendication 3,
**caractérisé en ce que** les moyens de mesure de la distance (D) comprennent une toise (T) qui s'étend parallèlement à l'axe du capteur (6), cette toise pouvant être amovible, dépliable et/ou télescopique,

5. Dispositif selon la revendication 3,
**caractérisé en ce que** les moyens permettant de mesurer la susdite distance (D) consistent en des moyens de télémétrie faisant intervenir un émetteurrécepteur et des moyens de comptage du temps que met une impulsion émise par l'émetteur pour parvenir au récepteur après réflexion sur la surface émissive.

6. Dispositif selon l'une des revendications précédentes,
**caractérisé en ce qu'**il comprend un capteur de température ambiante (13) relié à des moyens de correction (9) du signal délivré par le capteur (6).

7. Dispositif selon l'une des revendications précédentes,
**caractérisé en ce que** les susdits moyens de projection sont distincts dudit capteur.

8. Dispositif selon l'une des revendications précédentes,
**caractérisé en ce que** le susdit capteur comprend un corps (C1), une optique focalisatrice (F1) comprenant un réflecteur parabolique, une cellule optoélectronique (Dl) placée au foyer de l'optique (F1), un premier capteur additionnel de température (CT) du corps (C1) ainsi qu'un système de maintien de la température du corps à la température ambiante, **en ce que** la source de rayonnement lumineux (SL) ainsi que les moyens de projection (O) sont disposés coaxialement au réflecteur parabolique (12) entre le capteur (6) et la surface émissive (1) et **en ce qu'**il comprend des moyens de formation d'un objet lumineux (L) disposé entre les moyens de projection (O) et la source lumineuse (SL), de manière à obtenir dans la susdite deuxième zone de la surface émissive une image de l'objet lumineux (L) qui est nette, lorsque la distance entre les moyens de projection et ladite surface émissive (1) correspond à une distance de mise au point prédéterminée.

9. Dispositif selon la revendication 8,
**caractérisé en ce que** le susdit système de maintien de la température du corps (C1) à la température ambiante comprend une microturbine et/ou une cellule à effet Peltier (PR) montée sur le corps de manière à pouvoir abaisser la température de ce dernier et **en ce que** la commande de cette cellule (PR) est assurée par un asservissement en fonction de l'écart entre la température détectée par le premier capteur additionnel (CT) et la température détectée par un deuxième capteur additionnel (13) placé en un endroit convenable pour mesurer la température ambiante.

10. Dispositif selon l'une des revendications précédentes,
**caractérisé en ce qu'**il comprend des moyens de lecture d'une information d'identification de l'objet ou du corps.

11. Dispositif selon la revendication 10,
**caractérisé en ce que** les susdits moyens de lecture sont conçus de manière à assurer la lecture d'une puce transpondeur de type RFID.

12. Dispositif selon l'une des revendications 10 et 11,
**caractérisé en ce qu'**il comprend un circuit émetteur permettant de transmettre à distance, à destination d'un récepteur des données relatives à la température, à l'identification de l'objet ou du corps et des données d'horodatage.

## Claims

1. Device for the remote measurement of the temperature of an object or of a living body, this device comprising a directional infrared sensor (6) having a cylindrical or slightly conical detection volume so as to detect the infrared radiation emitted exclusively by a first zone (1) of the emissive surface of the object or body whose temperature it is wished to measure, which is included in said detection volume, means of processing the information delivered by the sensor (6), calculation means able to determine the temperature of said object or said body from said information, as well as a light-radiation source (SL) able to emit in the visible spectrum, this source (SL) being associated with projection means generating a light beam able to illuminate a second zone of the emissive surface including the first zone (1),
**characterised in that** it comprises a directional transmitter able to radiate in a precise wavelength range in the vicinity of the axis of the sensor (6) and means for determining a parameter relating to the attenuation undergone by this radiation during its path towards the emissive zone (1), its reflection on said zone (1) and its return to the reception part of the transmitter, the calculation means being designed so as to calculate the correction to be made to the temperature value measured by the sensor as a function of the parameter thus determined.

2. Device according to claim 1, **characterised in that** the aforementioned light source (SL) is of the pulse type.

3. Device according to one of claims 1 and 2, **characterised in that** it comprises means for measuring the distance (D) between the sensor (6) and the emissive zone (ZE) as well as temperature calculation means taking account of this distance (D).

4. Device according to claim 3, **characterised in that** the means of measuring the distance (D) comprises a gauge (T) that extends parallel to the axis of the sensor (6), this gauge being able be removable, foldable and/or telescopic.

5. Device according to claim 3, **characterised in that** the means for measuring the aforementioned distance (D) consist of telemetry means involving a transceiver and time-counting means that uses a pulse emitted by the emitter to reach the receiver after reflection on the emissive surface.

6. Device according to one of the preceding claims, **characterised in that** it comprises an ambient temperature sensor (13) connected to means of correcting (9) the signal delivered by the sensor (6).

7. Device according to one of the preceding claims **characterised in that** the aforementioned projection means are distinct from said sensor.

8. Device according to one of the preceding claims, **characterised in that** the aforementioned sensor comprises a body (C1), a focussing lens (F1) comprising a parabolic reflector, an optoelectronic cell (D1) placed at the focus of the lens (F1), a first additional temperature sensor (CT) for the body (C1) as well as a system for maintaining the temperature of the body at ambient temperature, **in that** the light-radiation source (SL) and the projection means (O) are disposed coaxially to the parabolic reflector (12) between the sensor (6) and the emissive surface (1) and **in that** it comprises means of forming a luminous object (L) disposed between the projection means (O) and the light source (SL), so as to obtain in the aforementioned second zone of the emissive surface an image of the luminous object (L) that is sharp, when the distance between the projection means and the said emissive surface (1) corresponds to a predetermined focussing distance.

9. Device according to claim 8, **characterised in that** the aforementioned system for maintaining the temperature of the body (C1) at ambient temperature comprises a microturbine and/or a Peltier effect cell (PR) mounted on the body so as to be able to reduce the temperature of the latter and **in that** control of this cell (PR) is provided by slaving as a function of the difference between the temperature detected by the first additional sensor (CT) and the temperature detected by a second additional sensor (13) placed at a suitable point for measuring the ambient temperature.

10. Device according to one of the preceding claims, **characterised in that** it comprises means of reading information identifying the object or body.

11. Device according to claim 10, **characterised in that** the aforementioned reading means are designed so as to provide the reading of a transponder chip of the RFID type.

12. Device according to one of claims 10 and 11, **characterised in that** it comprises an emitting circuit for remotely transmitting, to a receiver, data relating to the temperature or identification of the object or body and time-stamping data.

## Patentansprüche

1. Vorrichtung zur Fernmessung der Temperatur eines Gegenstands oder eines lebenden Körpers, und zwar folgendes umfassend: einen direktionalen Infrarot-Innenraumsensor (6), der ein zylindrisches oder leicht konisches Detektionsvolumen aufweist, um die Infrarotstrahlung zu ermitteln, die ausschließlich für einen ersten Bereich (1) der emissiven Oberfläche des Gegenstandes oder des Körpers ausgegeben wurde, an dem man die Temperatur messen will, was im genannten Detektionsvolumen eingeschlossen ist; Mittel zur Verarbeitung der durch den Sensor (6) ausgegebenen Informationen; Berechnungsmittel, die dazu geeignet sind, die Temperatur des genannten Gegenstandes oder des genannten Körpers anhand der besagten Informationen zu bestimmen, sowie eine Lichtstrahlquelle (SL) die dazu fähig ist, eine Strahlung im sichtbaren Spektrum abzugeben, wobei diese Quelle (SL) Projektionsmitteln zugeordnet ist, die einen Lichtstrahl erzeugen, der fähig ist, einen zweiten Bereich der emissiven Oberfläche, einschliesslich des ersten Bereiches (1) zu bestrahlen, **gekennzeichnet dadurch, dass** die besagte Vorrichtung einen direktionalen Sender umfasst, der dazu fähig ist, in einem präzisen Wellenlängenbereich in der Nähe der Achse des Sensors (6) zu strahlen, und Mittel, wodurch es möglich ist, einen Parameter bezüglich der Abschwächung zu bestimmen, die dieser Strahl im Verlauf seiner Strecke zur emissiven Zone (1) von seiner Rückstrahlung auf besagte Zone (1) und von seiner Umkehr zum Empfangsteil des Senders erfährt, wobei die Berechnungsmittel so konzipiert sind, um die Korrektur zu berechnen, die zu dem durch den Sensor gemessenen Temperaturwert nach dem so bestimmten Parameter zu erbringen ist.

2. Vorrichtung nach Anspruch 1, **gekennzeichnet dadurch, dass** die besagte Lichtquelle (SL) eine Impuls-Lichtquelle ist.

3. Vorrichtung nach einem der Ansprüche 1 und 2, **gekennzeichnet dadurch, dass** sie Mittel umfasst, wodurch es möglich ist, die Entfernung (D) zwischen dem Sensor (6) und der emissiven Zone (ZE) zu messen sowie Temperaturberechnungsmittel unter Berücksichtigung dieser Entfernung (D).

4. Vorrichtung nach Anspruch 3, **gekennzeichnet dadurch, dass** die Mittel zur Messung der Entfernung (D) einen senkrechten Messstab (T) umfassen, der sich parallel zur Achse des Sensors (6) erstreckt, wobei dieser Messstab abnehmbar, herunterklappbar und/oder teleskopisch sein kann.

5. Vorrichtung nach Anspruch 3, **gekennzeichnet dadurch, dass** die Mittel es ermöglichen, die besagte Entfernung (D) zu messen und aus Entfernungsmessmitteln bestehen, die einen Sender-Empfänger und Zeitzählungsmittel intervenieren lassen, die einen Impuls setzen, der vom Sender abgegeben wurde um zum Empfänger zu gelangen nach der Rückstrahlung auf die emissive Oberfläche.

6. Vorrichtung nach einem der vorangehenden Ansprüche, **gekennzeichnet dadurch, dass** sie einen Raumtemperatursensor (13) umfasst, der mit Korrektionsmitteln (9) des vom Sensor (6) ausgesandten Signals verbunden ist.

7. Vorrichtung nach einem der vorangehenden Ansprüche, **gekennzeichnet dadurch, dass** die obengenannten Projektionsmittel gesondert vom genannten Sensor sind.

8. Vorrichtung nach einem der vorangehenden Ansprüche, **gekennzeichnet dadurch, dass** der besagte Sensor einen Körper (C1) umfasst sowie eine Fokussierungsoptik (F1) mit einem parabolischen Reflektor, einer optoelektronischen Zelle (DI), die am Brennpunkt der Optik (F1) plaziert ist, einen ersten zusätzlichen Temperatursensor (CT) des Körpers (Cl) sowie ein System zur Beibehaltung der Körpertemperatur bei Raumtemperatur, **dadurch**, dass die Lichtstrahlquelle (SL) sowie die Projektionsmittel (O) koaxial am parabolischen Reflektor (12) zwischen dem Sensor (6) und der emissiven Oberfläche (1) angeordnet sind und dass sie Mittel zur Bildung eines Lichtgegenstands (L) umfassen, die zwischen den Projektionsmitteln (O) und der Lichtquelle (SL) angeordnet sind, um in der besagten zweiten Zone der emissiven Oberfläche ein Bild des Lichtgegenstandes (L) zu erhalten, das klar ist, wenn die Entfernung zwischen den Projektionsmitteln und der besagten emissiven Oberfläche (1) einem vorgegebenen Brennpunkt entspricht.

9. Vorrichtung nach Anspruch 8, **gekennzeichnet dadurch, dass** das besagte System zur Beibehaltung der Körpertemperatur (C1) bei Raumtemperatur eine Microturbine umfasst und/oder eine Zelle mit Peltier-Wirkung (PR), die auf dem Körper montiert ist, um die Temperatur von letzterem senken zu können und **dadurch**, dass die Schaltung dieser Zelle (PR) durch ein Steuergerät gewährleistet ist in Funktion des Abstandes zwischen der durch den ersten zusätzlichen Sensor (CT) ermittelten Temperatur und der Temperatur, die durch einen zweiten zusätzlichen Sensor (13) ermittelt wurde, der an einem zweckmäßigen Ort plaziert ist, um die Raumtemperatur zu messen.

10. Vorrichtung nach einem der vorangehenden Ansprüchen, **gekennzeichnet dadurch, dass** sie Lesemittel von einer Information über Identifizierung des Gegenstandes oder des Körpers umfasst.

11. Vorrichtung nach Anspruch 10, **gekennzeichnet dadurch, dass** die besagten Lesemittel so konzipiert sind, um das Ablesen von einem Mikrochip Transponder der Art RFID zu gewähren.

12. Vorrichtung nach einem der Ansprüche 10 und 11, **gekennzeichnet dadurch, dass** sie eine Senderschaltung umfasst, wodurch eine Fernübertragung zum Bestimmungsort eines Empfängers von Daten bezüglich der Temperatur, der Identifizierung des Gegenstandes oder des Körpers und Daten über die Zeitstempelung ermöglicht ist.
